# EUROPEAN PATENT APPLICATION

(11) **EP 4 179 874 A1**
(43) Date of publication of application: **17.05.2023**
(21) Application number: 21208640.9
(22) Date of filing: 16.11.2021
(51) Int. Cl.: A01N 33/18, A01N 35/06, A01N 43/42, A01N 43/84, A01N 43/90, A01N 59/12, A01P 1/00, D06M 10/04, D06M 16/00, A01N 25/10, A01N 25/34, A01N 25/26

(54) **ANTIMICROBIAL PHOTOACTIVE NANOFIBROUS POLYMER MATERIAL**

(71) Applicant: LAM-X a.s., 11000 Praha 1 (CZ)
(72) Inventor: Mosinger, Jiri, 14100 Praha 4 (CZ); Liska, Vojtech, 15500 Praha 13 (CZ); Krtenova, Petra, 10100 Praha 10 (CZ); Henke, Petr, 25101 Ricany (CZ); Chaloupka, Roman, 27201 Kladno (CZ)
(74) Representative: Harber IP s.r.o.

(57) **Abstract**

The invention provides an antimicrobial photoactive nanofibrous polymer material which comprises
- polymer nanofibers comprising hydrophobic domains and hydrophilic domains;
- at least one photoactive molecule encapsulated in the hydrophobic domains of the polymer nanofibers, said photoactive molecule being capable of releasing or generating antimicrobially active substance after irradiation by visible light.

The material of the invention may be used for antimicrobial wound dressings, antimicrobial cosmetic facial masks, self-disinfecting face masks or respirators, self-disinfecting filters for filtration of gases or liquids, self-disinfecting textile and products made thereof, self-disinfecting packaging material or protective agriculture foils.

## Description

### Field of Art

The present invention relates to an antimicrobial photoactive nanofibrous polymer material.

### Background Art

With an increasing incidence of viral and bacterial infections, often caused by resistant strains, and more generally also in connection with the problem of so-called nosocomial infections, which are a global health problem, research into new microbicidal methods is gaining in importance. The use of special photoactive dyes, so-called photosensitizers, producing singlet oxygen (O₂ (¹Δ_{g})) in photosensitized reactions, is therefore of great interest, mainly due to high reactivity and associated cytotoxicity of O₂ (¹Δ_{g}). This cytotoxicity is used in the so-called photodynamic inactivation of bacteria, viruses, yeasts and protozoa (Photodynamic Inactivation, PDI). A significant advantage of PDI is that the pathogens did not develop resistance against singlet oxygen.

Photosensitized generation of singlet oxygen O₂ (¹Δ_{g}) usually occurs in a liquid phase, where the photosensitizer is dissolved. However, this does not allow easy separation of the photosensitizer from the treated object or products after the photoreaction. When the photosensitizer is anchored to a solid support, it can be easily separated from the reaction products and reused. Furthermore, the desired effect can be achieved with a smaller amount or a lower concentration of the photosensitizer in the carrier as the surface area of the carrier increases. By encapsulating hydrophobic photosensitizers in polymeric nanofibers, the specific properties of photogenerated O₂ (¹Δ_{g}) (oxidative, cytotoxic effect, "in situ" activity) are combined with the specific properties of nanofiber membranes from suitable polymers (transparency to light, easy oxygen diffusion, large specific surface area). The very nanoporous structure of nanofiber membranes allows efficient capture of bacteria, viruses and other pathogens. The diffusion path of O₂ (¹Δ_{g}) is in the order of a maximum of hundreds of nanometers.

In previous studies, it has been found that nanofiber polymer membranes with encapsulated photosensitizers can be prepared by the "electrospinning" method. These membranes with a thickness of about 0.03 mm, area weight of about 2 g/m² and an average diameter of nanofibers within the range of 100-400 nm with encapsulated photosensitizer were characterized by UV-VIS, fluorescence and time-resolved spectroscopy (Mosinger, J., Jirsák, O., Kubát, P., Lang, K., Mosinger Jr., B. Bactericidal nanofabrics based on photoproduction of singlet oxygen Journal of Materials Chemistry, 2007, vol. 17 (2), p. 164-166).

A number of parameters may affect the properties, effectiveness, undesired quenching of the materials. For efficient use in various practical applications, there is still need to identify and provide suitable parameters allowing to provide a cost-effective, useful material with minimum or no undesired effects or properties.

For applications requiring disinfection of the wider membrane environment, it is possible to combine photogeneration of singlet oxygen with (photo) generation of other antimicrobial substances, e. g. NO or I₃⁻/I₂ with longer diffusion path. First experiments relating to these combinations were published in Dolansky, J., Henke, P., Kubát, P., Fraix, A., Sortino, S., Mosinger, J. Polystyrene Nanofiber Materials for Visible-Light-Driven Dual Antibacterial Action via Simultaneous Photogeneration of NO and O2(1Δg) ACS Applied Materials and Interfaces, 2015, vol. 7 (41), p. 22980-22989; Plí 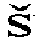 til, L. Henke, P., Kubát, P., Mosinger, J. Anion exchange nanofiber materials activated by daylight with a dual antibacterial effect Photochemical and Photobiological Sciences, 2014, vol. 13 (9), p. 1321-1329. However, such combinations only increase the number of problems which the practical use of such technology faces.

The existing photoactive polymer materials (see CZ303243B6) are sub-optimal, inter alia, they require a relatively long exposure time in order to excite sufficient antimicrobial activity, which may prevent or significantly limit the use of these materials in practice.

### Disclosure of the Invention

The inventors have defined the requirements for photoactive nanofibrous polymeric material, which would be applicable in practice. The requirements are very extensive and often contradictory. The basic requirements imposed on individual components from which the material is made of include, but are not limited to the following: The polymer should have a high oxygen permeability and nanofibers formed by the polymer should have a small diameter. The polymer should be transparent to light, nonpolar, but its surface needs to be wettable. The polymer composition must not adversely affect the photophysical characteristics of photosensitizers, especially in terms of quenching the triplet states of photosensitizer and/or singlet oxygen. The encapsulated photosensitizer should be hydrophobic, have a high quantum yield of singlet oxygen production and be photostable in the polymer environment, while the solvent must dissolve the polymer and at the same time the photosensitizer without aggregating or suffering chemical modification. Finally, the end material formed from these components by the spinning process must allow efficient capture of pathogens, should be homogeneous and have good mechanical properties. Setting all these parameters so that the resulting material optimally meets all the requirements is a highly complex and demanding multidimensional problem.

The aim of the present invention is to provide a solution to this complex problem and thus achieve a high efficacy of the antimicrobial action, i. e. to provide photoactive polymer nanofiber materials which would be suitable for real practical use, could be produced in a cost-effective manner, and would comply with all requirements for safety and effectiveness. A key prerequisite for antimicrobial efficacy is high singlet oxygen production (and/or nitric oxide radical production) which depends, inter alia, on the light dose/exposure. However, the same light exposure leading to the same production of singlet oxygen (and/or nitric oxide radical production) does not always result in the same antimicrobial activity. A light dose incident on the surface of the photoactive material in a shorter time results in the same amount of singlet oxygen as the same light dose incident on the surface of the photoactive material in a longer time period, but the antimicrobial activity produced in the shorter time period is higher. Therefore, one of the requirements enabling practical use of the photoactive antimicrobial material is high efficacy or yield of singlet oxygen photogeneration process, in order to allow to reach the critical threshold concentration of the antimicrobially active agent to achieve the bactericidal or virucidal effect. The present invention provides materials having these required properties.

The present invention provides an antimicrobial photoactive nanofibrous polymer material which comprises:
- polymer nanofibers comprising hydrophobic domains and hydrophilic domains;
- at least one photoactive molecule encapsulated in the hydrophobic domains of the polymer nanofibers, said photoactive molecule being capable of releasing or generating antimicrobially active substance after irradiation by visible light.

The encapsulated photoactive molecules can be selected from: at least one singlet-oxygen-producing photosensitizer, at least one nitric oxide (NO) radical photodonor and combinations thereof. For a singlet-oxygen-producing photosensitizer, the generated and/or released antimicrobially active substance are singlet oxygen molecules. For an NO radical photodonor, the generated and/or released antimicrobially active substance are nitric oxide radicals.

In some embodiments, the material comprising at least one singlet-oxygen-producing photosensitizer further comprises an iodide in the form of inorganic or organic iodide. The presence of iodide results in indirect photogeneration of I₃⁻/I₂ mediated by the singlet oxygen photogenerated by the photosensitizer. The iodide acts as an indirect photodonor of I₃⁻/I₂, another strong antimicrobial agent having a long diffusion length.

The term "nanofiber" or "nanofibers" herein, and unless indicated otherwise by the immediate context, refers to fibers having a diameter within the range of 10 to 950 nm, preferably 50 to 500 nm, and even more preferably from 100 to 350 nm. Generally, nanofibers may be produced by a variety of methods, including: electrospinning, centrifugal spinning, drawing, thermal-induced phase separation and template synthesis. Electrospinning and/or centrifugal spinning are preferred methods of production of nanofibers in this invention.

In general, the nanofibers may preferably be produced from polymers having number average molecular weight (Mn) within the range of 20 000 and 1 000 000, preferably between 30 000 and 900 000, and even more preferably from 40 000 to 700 000.

The terms "hydrophilic domains" and "hydrophobic domains" refer to parts of polymer chains having a hydrophilic or hydrophobic character, respectively. Each domain has a length of at least ten atoms involved in the linear polymer chain. The hydrophilic and hydrophobic domains of the nanofibers are formed by hydrophilic and hydrophobic domains of individual polymer chain(s), or by a mixture of hydrophilic and hydrophobic polymer, or by a mixture of a hydrophilic or hydrophobic polymer with a polymer having both hydrophobic and hydrophilic domains.

The term "polymer" includes homopolymers as well as copolymers. Copolymers include, for example, block copolymers, alternating copolymers and random copolymers.

The polymer nanofibers can be formed by one polymer or by a plurality of polymers. When the polymer nanofibers are formed by one polymer, this polymer contains both hydrophilic domains and hydrophobic domains in its chain (and is typically a copolymer). When the polymer nanofibers are formed by a plurality of polymers, these polymers may be hydrophilic polymers (including hydrophilic homopolymers and hydrophilic copolymers), hydrophobic polymers (including hydrophobic homopolymers and hydrophobic copolymers), or polymers containing both hydrophilic domains and hydrophobic domains in its chain.

The hydrophobic and hydrophilic domains of the nanofibers may be formed by the domains of one polymer. Such polymer would be a copolymer having hydrophilic and hydrophobic domains. Such copolymers may preferably include polyurethanes combining hydrophobic and hydrophilic domains or copolymers of caprolactone with a hydrophilic monomer. More preferably, such polymers include poly(ethylene oxide)/poly(ε-caprolactone) copolymers (PEO-PCL), polyurethanes comprising hexamethylenediisocyanate in combination with 1,4-butanediol as a chain extender and poly(ethylene oxide), and polyurethanes comprising linear polycarbonated diol, isophorone diisocyanate and isophorone diamine.

The hydrophobic and hydrophilic domains of the nanofibers may also be formed by at least one hydrophilic polymer and at least one hydrophobic polymer. These polymers are combined (e.g., mixed) and subjected to treatment to form nanofibers, such as electrospinning.

Suitable hydrophilic polymers preferably include poly(ethylene oxide) (PEO), polyvinylalcohol (PVA), , polyvinylpyrrolidone (PVP), cellulose esters and/or ethers, such as hydroxypropyl methylcellulose (HPMC), hydroxypropyl cellulose (HPC), cellulose acetate (CAc), chitosan, modified chitosans such as quarternised chitosans, N alkyl chitosans, carboxyalkyl chitosans, acyl chitosans, thiolated, sulfated and/or phosphorylated chitosans, polyacrylamide, polyacrylic acid, poly(N-isopropylacrylamide) (PNIPAA). More preferably, the hydrophilic polymers may be selected from the group including poly(ethylene oxide), cellulose acetate, hydroxypropyl methylcellulose, hydroxypropyl cellulose and polyacrylic acid.

Suitable hydrophobic polymers preferably include polyvinylbutyral (PVB), polyvinylidenefluoride (PVDF), polystyrene (PS), poly(vinylidene fluoride-co-hexafluoropropylene) (PVDF-HFP), poly(vinylidene fluoride-co-trifluoroethylene), poly(chlorotrifluoroethylene-co-vinylidene fluoride),polydimethylsiloxane (PDMS), polycaprolacton (PCL), polypropylene (PP), polytetrafluorethylene (PTFE), polymethylmethacrylate (PMMA), polycarbonate, polyamide 6. More preferably, polyvinylidenefluoride, poly(vinylidene fluoride-co-hexafluoropropylene), polydimethylsiloxane, polycaprolactone and polystyrene.

Even more preferably, the combination of hydrophilic and hydrophobic polymer is a combination of poly(ethylene oxide) and/or cellulose acetate from the group of hydrophilic polymers with one or more hydrophobic polymers selected from polyvinylidenefluoride, polystyrene, poly(vinylidene fluoride-co-hexafluoropropylene) or polycaprolactone.

In some embodiments, the nanofibers may comprise at least one copolymer having hydrophilic and hydrophobic domains, in combination with at least one hydrophilic polymer and/or at least one hydrophobic polymer. Preferably, copolymers of polyvinylidene fluoride may be combined with polyurethanes and/or copolymers of poly(ε-caprolactone). More specifically, poly(vinylidene fluoride-co-hexafluoropropylene), poly(vinylidene fluoride-co-trifluoroethylene) and/or poly(chlorotrifluoroethylene-co-vinylidene fluoride) can be combined with PEO/PCL copolymers and/or polyurethanes such as polyurethane having monomeric units derived from hexamethylenediisocyanate, 1,4-butanediol and poly(ethylene oxide), or polyurethane having monomeric units derived from linear polycarbonated diol, isophorone diisocyanate, and isophorone diamine.

The provision of hydrophilic and hydrophobic domains results in a stronger interaction between the nanofibers (in particular, of their hydrophobic domains) with the encapsulated photosensitizer(s) and/or NO radical photodonor(s). This reduces undesirable washing out of the encapsulated substances and prevents aggregation of the photoactive molecules which might result in lower photoactivity and consequently, lower antimicrobial activity. The presence of the hydrophilic domains helps to achieve dynamic surface wettability and, consequently, a better contact of nanofibers with the target microorganisms. Therefore, combination of hydrophilicity and hydrophobicity results in better overall antimicrobial activity of the material. Moreover, it may also improve its mechanical properties.

In preferred embodiments, the nanofibrous material has a translucence of at least 60 % for visible light (more specifically, for at least one wavelength in the range from 400 nm to 900 nm). In particular, the material has the translucence of at least 60 % in the visible light region of a significant absorption band of the selected photosensitizer(s) and/or NO photodonor(s) encapsulated in the polymer nanofibers. The translucence is typically determined on the basis of transmittance measurements in the visible light region using a standard two-beam UV-vis spectrometer on samples of polymeric nanomembranes with thickness of 0.03 mm in a cuvette filled with distilled water, the same solution in a second cuvette is used as reference.

The term "visible light" in this patent application refers to electromagnetic radiation having the wavelength within the range of visible and near infrared radiation spectrum, i.e. from 400 nm to 900 nm.

Suitable ratio between hydrophilic and hydrophobic domains depends on the nature of the particular polymers and in particular on the degree of their hydrophobicity or hydrophilicity. In general in this patent application, a polymer domain or a polymer is considered hydrophilic if the contact angle measured on the nanofiber layer made therefrom is lower than 5 degrees; a polymer domain or a polymer is considered highly hydrophobic if the contact angle measured on the nanofiber layer made therefrom is larger than 100 degrees and slightly hydrophobic if the contact angle is between 5 and 100 degrees. I.e., a hydrophobic polymer is a polymer with the contact angle measured on the nanofiber layer made therefrom higher than or equal to 5 degrees. In the simple case of nanofibers made of hydrophilic polymer(s) and hydrophobic polymer(s), a preferred ratio between hydrophilic polymer(s) and hydrophobic polymer(s) may range from 9:1 (w/w) to 1:20 (w/w). In the case of block copolymers, the preferred ratio between hydrophilic and hydrophobic domains of the block copolymer(s) may also range from 9:1 (w/w) to 1:20 (w/w). However, in case of other copolymers combining hydrophilic and hydrophobic domains in one polymer chain the optimal ratio between hydrophilic and hydrophobic domains depends on the individual polymer. It may be only roughly estimated since the contact angles for nanofibers made purely of hydrophobic or hydrophilic domains cannot be experimentally determined. In this case, it is preferred that the resulting surface of the nanofibers is hydrophilic (contact angle lower than 5 degrees), while both hydrophilic and hydrophobic domains are present in the polymer chain. Similarly in case of a more complex combination of polymers and copolymers (e.g. copolymer with domains of both types combined with a hydrophilic or hydrophobic polymer or copolymer), the optimal ratio between hydrophilic and hydrophobic domains and/or polymers depends on the individual polymers and copolymer(s) used. In this case, it is preferred that the resulting surface of the nanofibers is hydrophilic (contact angle lower than 5 degrees), while both hydrophilic and hydrophobic domains are present in the polymer chain.

A photosensitizer is generally a molecule which is capable of absorbing energy from light and utilizing the energy to cause a change in a neighboring molecule. The photosensitizer then returns to its ground state and remains chemically intact until it absorbs a further quantum of energy from light. Singlet-oxygen-producing photosensitizers are photosensitizers which are capable of converting molecular oxygen into singlet oxygen O₂ (¹Δ_{g}). The term "photosensitizer" in this patent application is intended to refer to "singlet-oxygen-producing photosensitizer".

Singlet-oxygen-producing photosensitizers include photoactive compounds like octahedral inorganic metal clusters, metal nanoparticles, silicon nanocrystals, metal organic frameworks, semiconductor structures, fullerenes or graphene/carbon and other types of quantum dots but preferably organic chromophores and their metal complexes. The photosensitizers of organic chromophore type may be preferably selected from the group of: aminoacridine dyes, phenothiazine dyes, phthalocyanine dyes, porphyrinoid dyes (porphyrins and similar macrocycles), xanthene dyes and endogenous organic molecules/photosensitizers originating from within a living system such as an organism, tissue, or cell (e.g. hematoporphyrins, chlorophylles, hypericins, and hypocrellins). More specifically, singlet-oxygen-producing photosensitizers include free base tetraphenylporphyrin (TPP), its zinc or magnesium derivative, zinc or aluminium phthalocyanine (ZnPc or AlPc), acridine orange (AO), toluidine blue (TB), crystal violet (CV), methylene blue (MB), malachite green (MG), rose bengal (RB), hypericin, hypocrellin A, naphthalocyanines with the absorption in the near infrared region. Even more specifically, free base tetraphenylporphyrin, its zinc or magnesium derivative, zinc or aluminium phthalocyanine, acridine orange, toluidine blue, crystal violet, methylene blue, malachite green, hypericin, naphthalocyanines with the absorption in the near infrared region. These photosensitizers are easily photoactivated by visible light matching their absorption bands. Singlet-oxygen-producing photosensitizers are encapsulated into polymer nanofibers, typically into their hydrophobic domains.

Nanofibers can be supplemented by a compound releasing, upon irradiation by visible light, a longer-lived antimicrobial compound with a longer diffusion length than singlet oxygen. NO radical photodonor or NO photodonor (both terms are used herein as synonyms) photoreleasing nitric oxide (NO) fits these requirements. This inorganic free radical has a lifetime of approximately 1 - 4 s in aqueous media and a diffusion distance of approximately 100 µm, whereas singlet oxygen has a lifetime of approximately 3.5 µs in aqueous media and a diffusion distance of approximately 200 nm. Analogously to singlet oxygen, NO is characterized by small size, absence of charge, multi-target therapeutic capability with a broad spectrum of antimicrobial activity limiting also formation of bacterial biofilm. Moreover, NO possesses vasodilatation properties and is known to be an endogenous regulator of inflammation. Its use to accelerate wound healing and cosmetic use are motivated by its role of the endogenous regulator. Preferably, the photogeneration of NO, exclusively triggered by visible light, can be achieved by encapsulation of nonpolar NO-photodonor into polymer nanofibers. Alternatively, the NO photodonor might be incorporated into polymer nanofibers by grafting or a charged NO photodonor might be bound by an electrostatic interaction to charged groups on the polymer chain.

NO radical photodonors to be encapsulated into polymer nanofibers (typically into their hydrophobic domains) include aromatic hydrocarbons typically with nitro- or nitroso- group, more specifically nitrobenzene derivatives with trifluoromethyl group in ortho position, derivates of N-nitrosoaminophenol, derivates of dimethylnitrobenzene or nitro-substituted polycyclic aromatic hydrocarbons. Even more specifically, the NO photodonors contain derivatives of: 1-amino-3-(trifluoromethyl)-4-nitrobenzenamine, 4-(N-(aminopropyl)-3-(trifluoromethyl)-4-nitrobenzenamine)-7-nitrobenzofurazan, dimethylnitrobenzene, 4-nitro-3-(trifluoromethyl)aniline, 6-nitrobenzo[a]pyrene, 1-[(2'-nitrophenyl)methoxy]-2-oxo-3,3-diethyl-1-triazene, 2,6-dimethylnitrobenzene, 4-(4,4-difluoro-2,6-diiodo-1,3,5,7-tetramethyl-4-bora-3a,4a-diaza-s-indacen-8-yl)-N-(3-((4-nitro-3-(trifluoromethyl)phenyl)(nitroso)amino)propyl)butanamide.

In some embodiments, nanofibers can be electrospun with iodide ions such as inorganic or organic iodides. In the presence of a photosensitizer photogenerating singlet oxygen, the iodide ion readily reacts with the singlet oxygen to form I₃⁻/I₂, another strong antimicrobial agent with a long diffusion length. The iodide in the polymer nanofiber matrix acts as an indirect photodonor of I₃⁻/I₂ since their release is achieved indirectly via photogeneration of O₂ (¹Δ_{g}). The singlet oxygen easily oxidizes I⁻ encapsulated in the nanofiber membrane to the corresponding antimicrobially active form of I₃⁻/I₂. The indirect photogeneration of I₃⁻/I₂ is exclusively controlled by visible light, irradiation time and irradiated area.

The singlet-oxygen-producing photosensitizer(s) inducing indirect I₃⁻/I₂ photogeneration preferably include free base tetraphenylporphyrin, its zinc or magnesium derivative, zinc or aluminium phthalocyanine, acridine orange, toluidine blue, crystal violet, methylene blue, malachite green, hypericin or a suitable combination thereof. The I₃⁻/I₂ indirect photodonors preferably include organic and inorganic iodide salts, more specifically, iodide salts of alkaline metals and alkaline earth metals, and quaternary ammonium iodides. Even more specifically, potassium iodide, sodium iodide, magnesium iodide and calcium iodide and tetraethylammonium iodide.

It is particularly advantageous to use a combination of several singlet-oxygen-producing photosensitizers, wherein the absorption spectra of the individual singlet-oxygen-producing photosensitizers overlap only partially, preferably overlap in less than 70 % of wavelength, more preferably overlap in less than 50 % of wavelength, even more preferably overlap in less than 30 % of wavelength, yet more preferably do not overlap.

It is also particularly advantageous to use a combination of several NO radical photodonors, wherein the absorption spectra of the individual NO radical photodonors overlap only partially, preferably overlap in less than 70 % of wavelength, more preferably overlap in less than 50 % of wavelength, even more preferably overlap in less than 30 % of wavelength, yet more preferably do not overlap.

The following combinations of two singlet-oxygen-producing photosensitizers may be preferred due to the small overlap of their absorption spectra: TPP & ZnPc, TPP & RB, TPP & MB, TPP & hypericin, TPP & MG, TPP & TBO, TPP & CV, AO & TBO, AO & MB, AO & CV and AO & MG.

When a combination of singlet-oxygen-producing photosensitizer(s) and NO radical photodonor(s), and optionally also I₃⁻/I₂ indirect photodonor(s) is used, it is advantageous if the absorption spectra of singlet-oxygen-producing photosensitizer(s) and NO radical photodonor(s) overlap only partially, preferably overlap in less than 70 % of wavelength, more preferably overlap in less than 50 % of wavelength, even more preferably overlap in less than 30 % of wavelength, yet more preferably do not overlap.

Based on the small or no overlap of absorption spectra of the photoactive compounds, the following singlet-oxygen-producing photosensitizer(s), and optionally iodide for I₃⁻/I₂ photogeneration, and NO radical photodonor(s) may be preferred: ZnPc, TBO, CV, MB, MG, chlorines, hypericin and naphthalocyanines; and optionally I₃⁻/I₂ indirect photodonors mentioned hereinbefore; and derivates of 1-amino-3-(trifluoromethyl)-4-nitrobenzenamine, 4-(N-(aminopropyl)-3-(trifluoromethyl)-4-nitrobenzenamine)-7-nitrobenzofurazan, dimethylnitrobenzene, 4-nitro-3-(trifluoromethyl)aniline or 6-nitrobenzo [a]pyrene.

In preferred embodiments, the polymer nanofibers should contain between 0.01 wt.% and 5 wt.% of the photosensitizer(s) and optionally from 0.1 wt.% to 10 wt.% of the relevant iodide, and/or from 0.1 wt.% to 10 wt.% of the NO photodonor . More preferably, from 0.05 wt.% to 1 wt.% of the photosensitizer(s) and optionally from 0.5 wt.% to 5 wt.% of the relevant iodide, and/or between 0.5 wt.% and 5 wt.% of the NO photodonor (wt.% = % w/w - relative to the weight of the polymer).

The term "encapsulated" refers to a state in which a molecule of the photoactive substance is entrapped to the hydrophobic domain(s) of polymeric nanofibers due to supramolecular interactions including for example weak hydrophobic interactions, van der Waals interactions and π-π interactions or other supramolecular interactions but not covalent interactions.

In some embodiments, the nanofibrous material further contains magnetic or magnetizable particles which are encapsulated in the nanofibers or adsorbed on the nanofibers. The magnetic or magnetizable particles may be microparticles (size 1 to 100 micrometers) or nanoparticles (size 5 to 1000 nanometers) on the basis of maghemite or magnetite stabilized by polyethyleneimine.

The nanofibrous material is in some embodiments placed on a base material or substrate, e.g., a layer of base material or substrate. The base material may be a polymeric material. Examples of base materials are: a polymer spunbond microfiber layer such as polypropylene spunbond, a polymer foam layer such as polyurethane foam, sticky tape, baking paper, silicon paper, wrapping film, aluminum foil and other easily available flat materials and/or substrates.

In a second aspect of the invention, a method for manufacturing of the material of the invention is provided. The method comprises the following steps:
- providing a solution or dispersion of a mixture of hydrophilic polymer(s) and hydrophobic polymer(s), and/or copolymer(s) with hydrophilic and hydrophobic domains in an appropriate solvent or in a mixture of suitable solvents;
- adding at least one singlet-oxygen-producing photosensitizer and optionally further at least one I₃⁻/I₂ indirect photodonor, and/or at least one NO radical photodonor to the solution or dispersion;
- producing nanofibers from the said solution or dispersion, preferably on a base material or substrate, preferably by electrospinning or centrifugal spinning;
- optionally subjecting the produced nanofibers to chemical or physical treatment.

Suitable solvents for the solution or dispersion of a mixture of hydrophilic polymer and hydrophobic polymer, and/or copolymer(s) with hydrophilic and hydrophobic domains are preferably selected from dimethylformamide (DMF), tetrahydrofuran (THF), acetic acid, formic acid, ethanol, methanol, chloroform, dimethylacetamide, dimethylsulfonamide (DMSO), acetone, distilled water, dichlormethane, toluene, isopropanol, hydrochloric acid, camphorsulfonic acid, trifluoroacetic acid, carbon disulfide, cyxlohexanone. It may be advantageous to use mixtures of these solvents such as: THF/DMF, chlorophorm/ethanol, chlorophorm/methanol, chlorophorm/ethanol/methanol, dimethylacetamide/acetone, ethanol/DMSO, acetone/DMSO, acetone/DMF and acetic acid/formic acid.

The presence of polymer nanofibers comprising hydrophobic domains and hydrophilic domains is crucial for good antimicrobial function of the photoactive material. As described hereinbefore, it can be achieved by combining polymer(s) with hydrophobic and hydrophilic domains. However, in principle the same result can be reached by electrospinning a hydrophobic polymer with the photoactive molecule(s) followed by a chemical or physical treatment of the electrospun nanofiber layer. It may include treatment by plasma, polydopamine, surfactants or sulfonation. These treatments represent an additional production step aimed at hydrophilizing nanofibers, especially their surface, but have significant disadvantages preventing the practical use of the photoactive materials thus obtained. In general, these procedures, together with the change in hydrophilicity, change or rather worsen other properties important for good antimicrobial function such as photoactivity of the encapsulated photoactive molecules, translucence of the polymer nanofibers and/or their mechanical properties.

A third aspect of the present invention are the nanofibrous materials of the present invention for use as antimicrobial wound dressings, antimicrobial cosmetic facial masks, self-disinfecting face masks or respirators, self-disinfecting filters for filtration of air (more generally of gases) or water (more generally liquids). The nanofibrous material may also be used as self-disinfecting textile, packaging material or protective agriculture foils. In all these applications, the photoactive antimicrobial nanofibrous polymer material enables an easy adaptation to the purpose of use, since it can be used either as a self-supporting layer or as another layer added to material commonly used for the relevant purpose mentioned hereinbefore.

### Brief Description of Drawings

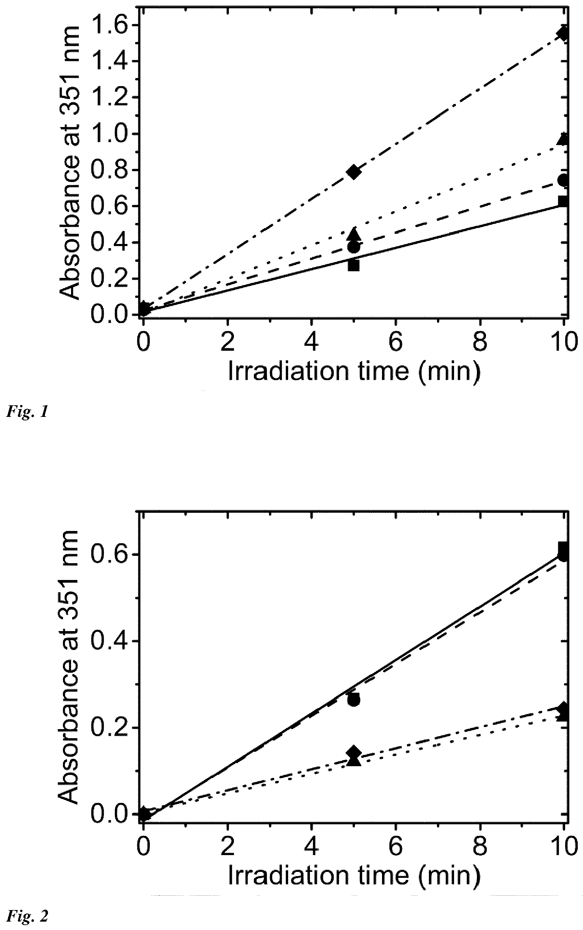

**Fig. 1****.** Effect of the hydrophilicity of the polymer matrix on the photoactivity of the materials measured through the formation of I₃⁻ induced by the photogenerated singlet oxygen. The UV/vis absorbance changes at 351 nm (attributed to the formation of I₃⁻ in the iodide detection solution) for different nanofiber materials containing polycaprolactone and poly(ethylene oxide) and 0.1% TPP photosensitizer were recorded at regular time intervals and compared to a blank solution of the same composition that was stored in dark. Gradual increase in the proportion of the hydrophilic component (PEO) in the PEO-PCL blend material from pure PCL nanofibers (squares/full line) through PCL:PEO blend 9:1 (circles/dashed line) and PCL:PEO blend 8:2 (triangles/dotted line) to PCL:PEO blend 7:3 (diamonds/dot-and-dash line) results in substantial increase in photoactivity demonstrating that the combination of hydrophobic and hydrophilic domains in the right proportions is crucial for photogeneration of singlet oxygen by the photoactive nanomaterial and its subsequent antimicrobial function.
**Fig. 2****.** Optimal proportion of hydrophilic and hydrophobic domains in polyurethanes results in an optimal photoactivity of the nanofiber material as measured through absorbance changes at 351 nm attributed to the formation of I₃⁻ induced by the photogenerated singlet oxygen in the iodide detection solution. The nanofibers electrospun from the polyurethanes having the ratio between the PEO segment and the rest of the polymer chain (w/w) of 3.3 (HH3 - squares/full line) or of 7.8 (HH8 - circles/dashed line) show high photoactivity (and consequently high photodynamic inactivation of microorganisms), whereas too hydrophilic polyurethane (with the ratio between the PEO segment and the rest of the polymer chain of 11.8 (HH12 - triangles/dotted line)) results in low photoactivity of nanofibers. If a polyurethane does not contain hydrophilic domains the photoactivity of nanofibers prepared in an analogous way is also significantly decreased (PU - diamonds/dot-and-dash line).

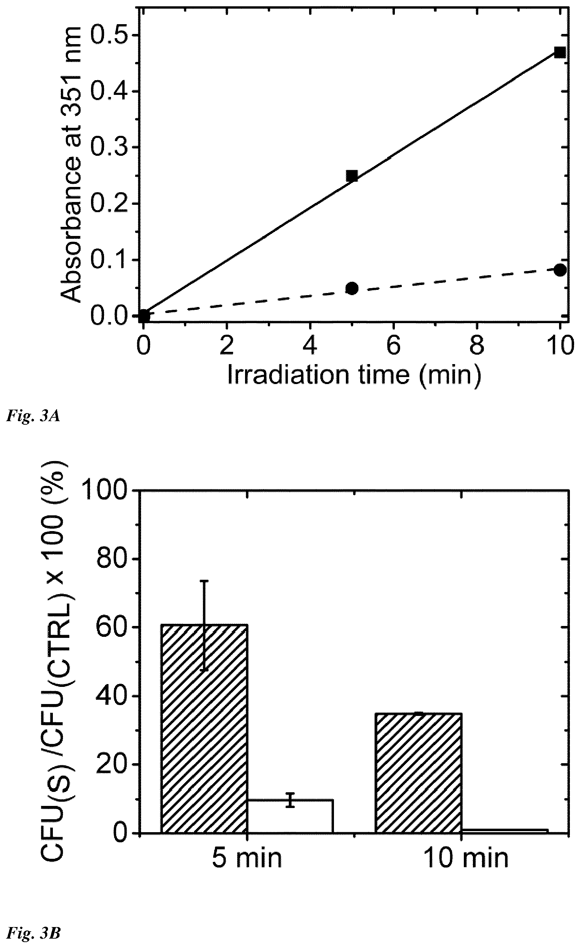

**Fig. 3****.** The presence of hydrophilic domains is crucial for optimal photoactivity of the nanofiber material (A) as well as for its antimicrobial activity (B). The nanofiber membrane electrospun from purely hydrophobic PVDF-HFP with encapsulated TPP (dashed line/striped columns) demonstrates several times lower photoactivity (A) and accordingly, also significantly lower photoinduced antimicrobial activity (B) than hydrophobic-hydrophilic mixture of PVDF-HFP and polyurethane having the ratio between the PEO segment and the rest of the polymer chain of 7.8 (HH8 - full line/white columns). Panel A shows absorbance changes at 351 nm attributed to the formation of I₃⁻ induced by the photogenerated singlet oxygen in the iodide detection solution at defined times after irradiating samples of materials with visible light, whereas panel B shows antimicrobial activity of photoactive materials depicted as number of colony-forming units (CFU) related to the number of CFU of control sample - equivalent nanofiber material without irradiation. It is possible to note that the photoactivity measured spectroscopically and the antimicrobial activity measured as a reduction of the number of CFU are naturally highly correlated.

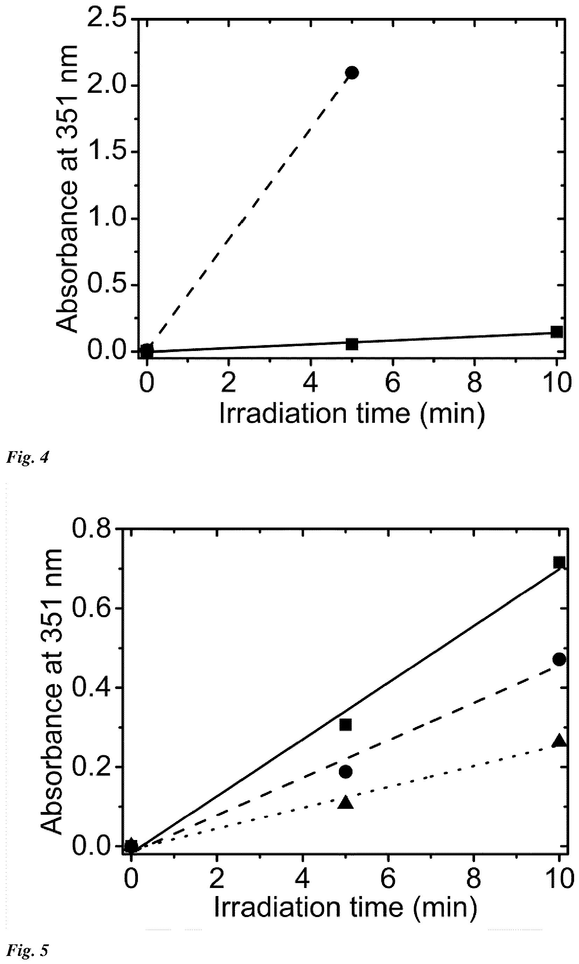

**Fig. 4****.** Comparison of PVB/HPC nanofibers with encapsulated 1% rose bengal (dashed line) to purely hydrophobic nanofibers made of PVB under the same experimental conditions (full line) show that the presence of hydrophilic domains is needed to improve significantly the photoactivity of nanofibers.
The individual points show absorbance changes at 351 nm attributed to the formation of I₃⁻ induced by the photogenerated singlet oxygen in the iodide detection solution at defined times after irradiating samples of materials with visible light.
**Fig. 5****.** When excited by white light, a synergistic effect of two encapsulated photosensitizers with no or low excitation spectra overlap can be observed. Comparison of three different nanofiber materials manufactured by electrospinning of 10% solutions of polyvinybutyral in ethanol supplemented by 0.5% RB (circles/dashed line) or 0.1% TPP (triangles/dotted line) or 0.5% RB & 0.1% TPP (squares/full line) demonstrates that TPP and RB cover larger region of the white light spectrum than individual photosensitizers and consequently, the singlet oxygen is produced with higher yield under the same light exposition. The individual points show absorbance changes at 351 nm attributed to the formation of I₃⁻ induced by the photogenerated singlet oxygen in the iodide detection solution at defined times after irradiating samples of the materials with visible light.

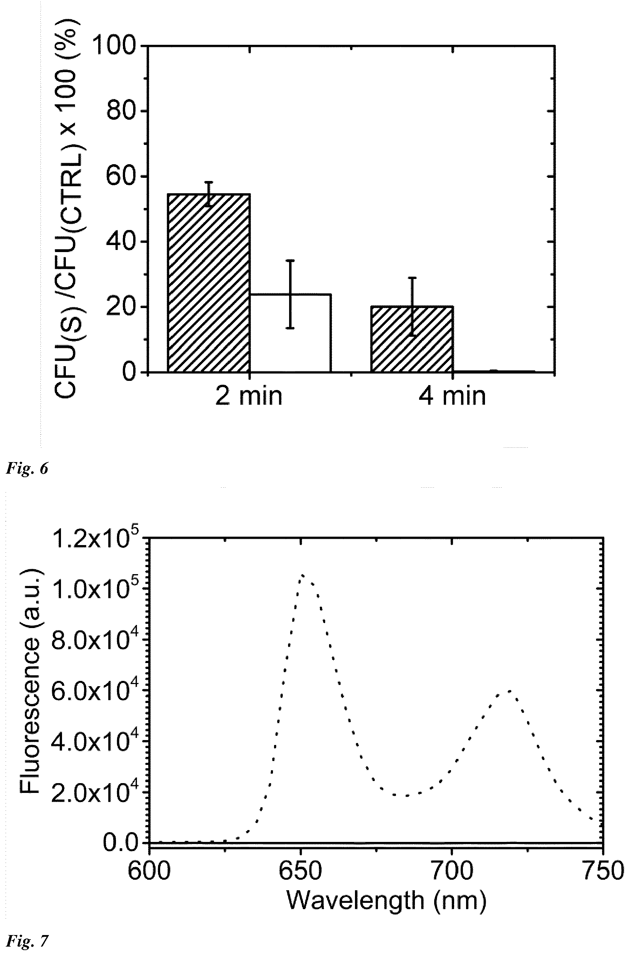

**Fig. 6****.** Photorelease of iodine triggered by photogenerated singlet oxygen can further increase the antimicrobial activity of the nanofiber material. Nanofiber materials electrospun from the same 16% solution of polystyrene and the polyurethane having the ratio between the PEO segment and the rest of the polymer chain (w/w) of 7.8 (HH8) with TPP in the absence (stripped columns) and in the presence of 10% potassium iodide (white columns) show remarkable difference in antimicrobial activity depicted as number of colony-forming units (CFU) related to the number of CFU of control sample - equivalent nanofiber material without irradiation.
**Fig. 7****.** Fluorescence emission spectra of nanofiber material leachate show that the photosensitizer does not leach out from the nanofiber materials with encapsulated photosensitizer molecules produced by electrospinning. A piece (4 cm²) of nanofiber membrane was shaken at 90 RPM either in 4 mL of THF (dotted line) or in 4 mL of distilled water (full line) at laboratory temperature for 12 hours.
Fluorescence emission spectra of these two samples excited at 420 nm show high fluorescence of TPP in the sample in THF (dotted line) since the polymer nanofibers and the encapsulated photosensitizer are completely dissolved in this solvent whereas no TPP fluorescence can be detected in an aqueous extract of this material (full line).

### Examples

### Example 1

In a 16 % solution of poly(ethylene oxide) (PEO) and polycaprolactone (PCL) 3:7 (w/w) in chloroform-ethanol mixture (8:2 w/w), TPP (or ZnPc) is dissolved at a concentration of 0.001 g photosensitizer per 1 g of polymer and processed by electrospinning to produce a nanofibrous layer deposited on the surface of a base material, e. g. polypropylene spunbond microfiber layer. The number average molecular weights of PEO and PCL are of 100 000 g/mol and 80 000 g/mol, respectively.

The same procedure was repeated with the ratios of PEO:PCL 2:8 and 1:9 w/w.

The hydrophilic polymer component (PEO) in PCL/PEO polymer blend nanofibers or in PEO-PCL copolymer nanofibers is responsible for introduction of a partially hydrophilic character into nanofibers. This enables more effective photogeneration of singlet oxygen than in case of nanomembrane electrospun from pure more hydrophobic PCL under the same conditions and consequently, results in a significantly higher antimicrobial activity of the mixed/amphiphilic nanofiber membrane.

Similarly, a PEO-PCL copolymer (1:1, Mn = 40 000 g/mol) is dissolved together with the same photosensitizer under the same conditions to obtain an analogous nanofiber layer. This co-polymer approach results also in an improvement of singlet oxygen photogeneration and of antimicrobial activity as compared to PCL itself.

### Example 2

In a 10 % solution of polyurethanes (Mn = 120 000 - 180 000 g/mol) in tetrahydrofuran/ dimethylformamide (THF/DMF) mixture (7:3 w/w) supplemented by 0.15% TEAB (% w/w - relative to the weight of the polymer), TPP is dissolved at a concentration of 0.01 g photosensitizer per 1 g of polymer and processed by electrospinning to produce a nanofibrous layer deposited on the surface of a base material, e.g. polypropylene spunbond microfiber layer. The nanofibers prepared in this way from polyurethanes whose hydrophilic segment consists of PEO and the more hydrophobic segment of hexamethylenediisocyanate in combination with 1,4-butanediol as a chain extender can have different hydrophobic to hydrophilic domain ratio depending on the amount of the individual segments in the alternating copolymer chain. This example demonstrates that only nanofibers thus produced from polyurethanes having optimal hydrophilic to hydrophobic domain ratio show sufficiently high photoactivity.

The nanofibers electrospun from the polyurethanes having the ratio between the PEO domain and the rest of the polymer chain (w/w) of 3.3 (HH3) or of 7.8 (HH8) show high photoactivity (and consequently high photodynamic inactivation of microorganisms), whereas too hydrophilic polyurethane (with the ratio between the PEO segment and the rest of the polymer chain of 11.8 (HH12) results in low photoactivity of nanofibers. If a polyurethane (PU) does not contain hydrophilic domains the photoactivity of nanofibers prepared in an analogous way is also significantly decreased. Therefore, taking into account the hydrophobic character of the photosensitizers, the photogeneration of singlet oxygen can be doubled by careful selection of the polymer composition with regard to a balance between hydrophilic and hydrophobic domains.

### Example 3

Hydrophilic or hydrophobic properties need not always be limited to a single polymer or polymer segment, it is possible to combine in a blend an amphiphilic co-polymer such as a polyurethane with the favorable ratio between hydrophilic and hydrophobic parts from the previous example with a purely hydrophobic polymer such as polystyrene or PVDF or a hydrophobic co-polymer such as poly(vinylidene fluoride-co-hexafluoropropylene). These complex combinations enable the improvement of other properties of the polymer nanofiber layer, e. g. the improvement of mechanical properties or of oxygen permeability. A 16% solution of polystyrene (Mw = 192 000 g/mol) and the polyurethane (1:1 w/w) having the ratio between the PEO segment and the rest of the polymer chain (w/w) of 7.8 (HH8) (Mn = 120 000 - 180 000 g/mol) described in Example 2 and 0.5% ZnPc in THF/DMF mixture (7:3 w/w) is used for electrospinning of polymer nanofibers deposited on the surface of a polypropylene spunbond microfiber layer. Similarly, a solution of PVDF-HFP (Mn = 130 000 g/mol) and this polyurethane (1:1 w/w) having the ratio between the PEO segment and the rest of the polymer chain (w/w) of 7.8 described in Example 2 and 1% TPP or hypericin in dimethylacetamide/acetone mixture (7:3 w/w) is used for electrospinning of polymer nanofibers. Photogeneration of singlet oxygen (and also photodynamic inactivation of microbes) is higher in complex polymer systems combining hydrophilic and hydrophobic polymer or parts of the polymer chain than in purely hydrophobic nanofibers manufactured under otherwise identical conditions from hydrophobic polymers such as polystyrene (Mw = 192 000 g/mol), PVDF (Mw = 156 000 g/mol) or PVDF-HFP (Mn = 130 000 g/mol) only.

### Example 4

In a 20% solution of polyvinybutyral and hydroxypropyl cellulose (7:3 w/w) in ethanol, rose bengal (methylene blue or or chlorophyll A or chlorine or hypericin) is dissolved at a concentration of 0.01 g photosensitizer per 1 g of polymer and processed by electrospinning to produce a nanofibrous layer deposited on the surface of a polypropylene spunbond microfiber layer. The number average molecular weights of PVB and HPC are of 200 000 g/mol and 240 000 g/mol, respectively. Photoactivity comparison of PVB/HPC nanofibers with purely hydrophobic nanofibers made of PVB under the same experimental conditions show that the presence of hydrophilic domains is needed to improve it significantly.

### Example 5

Under conditions of the same exposure to light photoactivating the antimicrobial function of nanofibers, the photoactivity can be further increased by encapsulating another photosensitizer with complementary excitation spectrum to the photosensitizer already encapsulated within the polymer nanofibers. Three different 10% solutions of polyvinybutyral in ethanol supplemented by 0.5% RB or 0.1% TPP or 0.5% RB & 0.1% TPP (% w/w - relative to the weight of the polymer) are processed by electrospinning to produce a nanofibrous layers deposited on the surface of a polypropylene spunbond microfiber layer. The number average molecular weight of PVB is of 200 000 g/mol. When excited by white light, a synergistic effect of two encapsulated photosensitizers can be observed. Under the same conditions, the nanofiber material with two encapsulated photosensitizes demonstrates higher photoactivity than any of the materials with encapsulated photosensitizer of only one type. TPP and RB absorption spectra do not overlap too much and cover larger region of the white light spectrum and consequently, the singlet oxygen is produced with higher yield under the same light exposition. Even higher efficacy can be achieved in polymer nanofibers combining hydrophobic and hydrophilic domains as described in the following example.

### Example 6

Similarly to the examples 1 and 5, a 16 % solution of cellulose acetate and polycaprolactone 3:7 (w/w) and 0.1 %solution of TPP in chloroform-ethanol-methanol mixture (8:1:1 w/w/w) is supplemented by 0.1 % hypericin as a second photosensitizer and processed by electrospinning to produce a nanofibrous layer deposited on the surface of a base material, e.g. polypropylene spunbond microfiber layer (% w/w - relative to the weight of the polymer). The number average molecular weights of cellulose acetate and PCL are of 50 000 g/mol and 45 000 g/mol, respectively. When excited by white light, this nanofiber material demonstrates higher yield of singlet oxygen generation and also higher antimicrobial activity than a similar material with photosensitizer of only one type, since both encapsulated photosensitizers, TPP with absorption in blue region of the visible spectrum and hypericin with predominant absorption in red spectral region, produce the singlet oxygen and contribute to the overall activity of the nanofibrous material.

### Example 7

A 16% solution of polystyrene (Mw = 192 000 g/mol) and the polyurethane having the ratio between the PEO segment and the rest of the polymer chain (w/w) of 7.8 (HH8) (Mn = 120 000 - 180 000 g/mol) described in Example 2 (PS:PU = 2:3 w/w) in THF/DMF mixture (7:3 w/w) is used for electrospinning of polymer nanofibers with 10% or without potassium iodide (% w/w - relative to the weight of the polymer).As in previous cases, the encapsulated photosensitizer generates singlet oxygen upon irradiation but in addition, the photogenerated singlet oxygen mediates the (photo)release of iodine and triiodide which in turn increase the antimicrobial activity of the nanofibrous material. The amount of released iodine or triiodide can be controlled by duration of the light exposure.

### Example 8

Similarly to example 5, the photoactivity of nanofibers (and also their antimicrobial activity) can be increased by encapsulating both, a photosensitizer and a NO photodonor with an excitation spectrum complementary to the photosensitizer, e.g. a 16% solution of polystyrene (Mw = 192 000 g/mol) and a polyurethane (Mn = 120 000 - 180 000 g/mol) (7:3) described in Example 7, 1 % photosensitizer (ZnPC) and 1 to 5% NO photodonor (N- (aminopropyl)-3-(trifluoromethyl)-4-nitrobenzenamine) in dimethylacetamide/acetone mixture (7:3 w/w) is used for electrospinning of polymer nanofibers on the surface of a base material (% w/w - relative to the weight of the polymer). The photorelease of NO radical has a strengthening effect on the antimicrobial efficacy of the material similar to the (photo)release of iodine and triiodide in the previous example.

### Example 9

A 16% solution of PVDF-HFP (Mn = 130 000 g/mol) and a polyurethane (Mn = 120 000 - 180 000 g/mol) (7:3 w/w) with the favorable ratio between hydrophilic and hydrophobic parts described in Example 7 and 1 to 5% NO photodonor (N- (aminopropyl)-3-(trifluoromethyl)-4-nitrobenzenamine) in dimethylacetamide/acetone mixture (7:3 w/w) is used for electrospinning of polymer nanofibers on the surface of a base material. The antimicrobial effect of the NO photoreleased from the nanofibrous material reaches a greater distance from the material than photogenerated singlet oxygen having only a short diffusion distance. The amount of released NO can be controlled by duration of the light exposure. Since the NO is known for a wider range of biological effects, the photorelease of NO from the material can also be used for other than just antimicrobial therapy.

### Example 10

The antimicrobial activity of nanofibrous materials can be further increased by combining photorelease of three different antimicrobial species in one polymer matrix. It means the photorelease of NO, the photogeneration of singlet oxygen and the photorelease of iodine and/or triiodide mediated by the generated singlet oxygen at the same time, however, the excitation spectra of NO photodonor and photosensitizer should not overlap. A 16 % solution of cellulose acetate and polycaprolactone 3:7 (w/w), 0.1 % solution of acridine orange, 0.1 % solution of potassium iodide and 1 % solution of N-(aminopropyl)-3-(trifluoromethyl)-4-nitrobenzenamine in chloroform-ethanol-methanol mixture (8:1:1 w/w/w) is processed by electrospinning to produce a nanofibrous layer deposited on the surface of a base material, e.g. polypropylene spunbond microfiber layer (% w/w - relative to the weight of the polymer).

### Example 11

During the electrospinning process, any of the antimicrobial photoactive polymer nanofiber materials mentioned in the preceding examples can be deposited on the base layer being simultaneously a common wound dressing material, e. g. a wound dressing made of polyurethane foam. The presence of this photoactive layer provides the composite / layered wound dressing with an additional function, it improves the healing of chronic or poorly healing wounds mainly by preventing secondary wound infection. Alternatively, the layered (or sandwich) wound dressing does not have to be assembled during the electrospinning process, but can be assembled subsequently from individual layers.

### Example 12

The nanofibrous polymer materials mentioned in the previous examples (1-10) are electrospun on a supporting layer, most often on a polypropylene spunbond, but may be produced on or combined with virtually any sheet material. The manufacturing process thus provides considerable variability in the use of the nanomaterial. The photoactive nanofibrous material with antimicrobial function can be used either in combination with another layer or as a self-supporting layer and consequently, enables an easy adaptation to the purpose of use. The material can be easily adapted and used not only as a wound cover, but also as self-disinfecting face masks or respirators, self-disinfecting filters for filtration of gases or water liquids. It may also be used as self-disinfecting textile or packaging material.

### Example 13 (comparative)

The comparison of electrospun polystyrene nanofibers with photosensitizer before and after postprocessing surface modifications (by sulfonation, cold plasma or polydopamine coating) clearly demonstrates that these modifications increase the wettability of originally completely hydrophobic surface of nanofiber membrane and significantly increases the contact of photogenerated singlet oxygen with chemical and/or biological targets on the surface of nanofiber membrane, and consequently also the antimicrobial activity of the membrane. However, these modifications are time consuming, moreover usually impairs the photophysical and/or mechanical properties of the nanofiber membrane. As example, the above mention sulfonation decrease the mechanical properties and oxygen diffusion, the plasma treatment can destroy the molecules of the photosensitizer on the surface of nanofibers and also negatively alter the oxygen diffusion, the grey polydopamine coating reduces the transparency of the material and limit the useful singlet oxygen pathway from the surface of nanofibers.

In a 17 % solution of polystyrene (Mw = 192 000 g/mol) in cyclohexanon supplemented by 0.07% TEAB (% w/w - relative to the weight of the polymer), TPP is dissolved at a concentration of 0.01 g photosensitizer per 1 g of polymer and processed by electrospinning to produce a nanofibrous layer deposited on the surface of a base material. The material is then modified by sulfonation or oxygen plasma treatment or polydopamine coating. For sulfonation, the nanofiber membrane is immersed in 96% sulfuric acid at room temperature for 2 hours and then washed with deionized water and neutralized with 25% ammonia hydroxide solution for 24 hours. For oxygen plasma treatment, the material is oxidized in the radio frequency oxygen plasma using a low-pressure FEMTO plasma system (Diener Electronic GmbH & Co. KG) pumped down to a basic pressure of 8 Pa by scroll vacuum pump. The oxidation is provided in pure O2 atmosphere at a pressure of 30 Pa with a power of 8 W during 20 s. For polydopamine coating, the nanofiber membrane is dipped in ethanol for 2 mins and then incubated in an aqueous solution of dopamine (2mg/ml in 10 mM Tris, pH 8.5) for 30 mins. The said surface modifications change the hydrophobic surface of the material into highly hydrophilic but this change has a negative impact on other important properties of the material.

### Example 14 (comparative)

If a photosensitizer (generally usually a hydrophobic molecule) is encapsulated in nanofibers of gelatin as an example of hydrophilic polymer, it results in an aggregation of hydrophobic photosensitizer molecules in the nanofiber membranes and therefore loss of its emission and photosensitization properties, since the photosensitization process requires thephotosensitizer to be in mostly monomeric state.

A 17% solution of gelatin in formic acid:acetic acid mixture (1:3) with TPP dissolved at a concentration of 0.01 g photosensitizer per 1 g of polymer is used for electrospinning of polymer nanofibers on the surface of a base material. Fluorescence emission spectra of the material measured immediately after electrospinning show high fluorescence of TPP upon excitation at 420 nm. Incubation of the material in humid environment above beaker containing water for 12 hours causes aggregation of the photosensitizer and thus almost complete loss of its emission and photooxidation properties.

### Example 15

A 16% solution of polycaprolactone (Mw = 45 000 g/mol) and cellulose acetate (Mw = 50 000 g/mol) 7:3 (w/w) in chroform:ethanol mixture (8:2) with TPP dissolved at a concentration of 0.001 g photosensitizer per 1 g of polymer is used for electrospinning of polymer nanofibers. A piece (4 cm²) of nanofiber membrane was shaken at 90 RPM either in 4 mL of THF or in 4 mL of distilled water at laboratory temperature for 12 hours. Fluorescence emission spectra of these two samples excited at 420 nm show high fluorescence of TPP in the sample in THF since the polymer nanofibers and the encapsulated photosensitizer are completely dissolved in this solvent whereas no TPP fluorescence can be detected in an aqueous extract of this material. Therefore, the photosensitizer molecules are firmly encapsulated in the polymer nanofibers and do not leach out.

### Material and methods

### Antibacterial Activity Testing

A culture of Escherichia coli DH5α (Invitrogen, CA) with plasmid pGEM11Z (Promega, WI) was incubated at 37 °C with stirring in LB medium (Carl Roth, Germany) containing 1% of ampicillin (Carl Roth, Germany). The incubation was terminated when the absorbance at 600 nm reached 0.8 (approximately 1.6 x 10⁸ CFU/ml). The culture was diluted 350x in PBS (Carl Roth, Germany). The nanofiber materials (1.5 x 1.5 cm) with encapsulated TPP were placed on a sterile cotton pad in a petri dish prewetted with 1500 µL of PBS. The surfaces of the materials were inoculated with 5 µL of the diluted bacterial suspension. After 5-min incubation, the materials were either irradiated by a 18 W blue (420 nm) LED light (Rubylux, USA) with a 400 nm longpass filter (for elimination of UV light (ThorLabs)) for 5 or 10 min or were stored in the dark. Then the samples were placed in Eppendorf tubes with 500 µL of PBS and vigorously shaked for 1 min (IKA vortex 3). After shaking, the membranes were removed and 150 µL of the bacterial suspension was placed on sterile agar plates in duplicates. The plates were incubated for 15 h in darkness at 37 °C for bacterial colony growth. The numbers of colony forming units (CFU) were then calculated using OpenCFU software (Quentin Geissmann 2012-2013).

Photodynamic inactivation was calculated as a proportion of the CFUs of E. *coli* observed on agar plates after inoculation with bacteria collected from the surfaces of irradiated samples and dark controls.

**Quantification of Photooxidation Process** (see J. Mosinger, B. Mosinger, Photodynamic Sensitizers Assay: Rapid and Sensitive Iodometric Measurement, Experientia, 51 (1995) 106-109. http://dx.doi.org/10.1007/BF01929349). Briefly, a piece of the nanofiber membranes (2 cm²) fixed on quartz glass was placed in a thermostated 10 mm quartz cell (22 °C) containing 3 ml of iodide detection solution. The following composition of iodide detection solution for O₂ (¹Δ_{g}) was used: 0.12 M KI, 10 µM (NH₄)₂MoO₄ in 0.02 M sodium-potassium phosphate buffer, pH 6.2. The cell was irradiated with visible light by using a stabilized xenon lamp (500 W, Newport) with a long-pass filter (λ ≥ 400 nm, Newport). The UV/vis absorbance changes at 351 nm, which are attributed to the formation of I₃⁻, were recorded at regular time intervals and compared to a blank solution of the same composition that was stored in dark. The overall light dose (for 10 min irradiation in the relevant spectral interval 400-700 nm based on lamp manufacturer data) and the estimated dose of absorbed light calculated from absorption spectra were 83 J/cm² and 8 J/cm², respectively.

**Contact angle measurement** (see Volpe, C. D.; Brugnara, M.; Maniglio, D.; Siboni, S.; Wangdu, T. (2006). "About the possibility of experimentally measuring an equilibrium contact angle and its theoretical and practical consequences". Contact Angle, Wettability and Adhesion. 4: 79-100)

## Claims

1. Antimicrobial photoactive nanofibrous polymer material which comprises
- polymer nanofibers comprising hydrophobic domains and hydrophilic domains;
- at least one photoactive molecule encapsulated in the hydrophobic domains of the polymer nanofibers, said photoactive molecule being capable of releasing or generating antimicrobially active substance after irradiation by visible light.

2. Material according to claim 1, wherein the photoactive molecule is selected from at least one singlet-oxygen-producing photosensitizer, at least one nitric oxide (NO) radical photodonor and combinations thereof.

3. Material according to claim 1, wherein the photoactive molecule includes singlet-oxygen-producing photosensitizer and the material further comprises an iodide ion in the form of inorganic or organic iodide.

4. Material according to any one of the preceding claims, wherein the polymer nanofibers are formed by
- at least one polymer containing both hydrophilic domains and hydrophobic domains in its chain;
- a combination of at least one polymer containing both hydrophilic domains and hydrophobic domains in its chain, and at least one hydrophilic polymer;
- a combination of at least one polymer containing both hydrophilic domains and hydrophobic domains in its chain, and at least one hydrophobic polymer;
- a combination of at least one hydrophobic polymer and at least one hydrophilic polymer;
- a combination of at least one polymer containing both hydrophilic domains and hydrophobic domains in its chain, and at least one hydrophilic polymer, and at least one hydrophobic polymer.

5. Material according to any one of the preceding claims wherein the polymer nanofibres contain at least one polymer containing both hydrophilic domains and hydrophobic domains in its chain which is selected from polyurethanes combining hydrophobic and hydrophilic domains or copolymers of caprolactone with a hydrophilic monomer; preferably it is selected from poly(ethylene oxide)/poly(ε-caprolactone) copolymers (PEO-PCL), polyurethanes comprising hexamethylenediisocyanate in combination with 1,4-butanediol as a chain extender and poly(ethylene oxide), and polyurethanes comprising linear polycarbonated diol, isophorone diisocyanate and isophorone diamine.

6. Material according to any one of the preceding claims wherein the polymer nanofibres contain at least one hydrophilic polymer which is selected from poly(ethylene oxide) (PEO), polyvinylalcohol (PVA), polyvinylpyrrolidone (PVP), cellulose esters and/or ethers, such as hydroxypropyl methylcellulose (HPMC), hydroxypropyl cellulose (HPC), cellulose acetate (CAc), modified chitosans such as quarternised chitosans, N alkyl chitosans, carboxyalkyl chitosans, acyl chitosans, thiolated, sulfated and/or phosphorylated chitosans polyacrylamide, polyacrylic acid, poly(N-isopropylacrylamide) (PNIPAA).

7. Material according to any one of the preceding claims wherein the polymer nanofibres contain at least one hydrophobic polymer which is selected from polyvinylbutyral (PVB), polyvinylidenefluoride (PVDF), polystyrene (PS), poly(vinylidene fluoride-co-hexafluoropropylene) (PVDF-HFP), poly(vinylidene fluoride-co-trifluoroethylene), poly(chlorotrifluoroethylene-co-vinylidene fluoride), polydimethylsiloxane (PDMS), polycaprolacton (PCL), polypropylene (PP), polytetrafluorethylene (PTFE), polymethylmethacrylate (PMMA), polycarbonate, polyamide 6.

8. Material according to any one of the preceding claims wherein the polymer nanofibres contain a combination of poly(ethylene oxide) and/or cellulose acetate from the group of hydrophilic polymers with one or more hydrophobic polymers selected from polyvinylidenefluoride, polystyrene, poly(vinylidene fluoride-co-hexafluoropropylene) or polycaprolactone; or a combination of polyvinylidene fluoride copolymers with polyurethanes and/or copolymers of poly(ε-caprolactone); or a combination of poly(vinylidene fluoride-co-hexafluoropropylene), poly(vinylidene fluoride-co-trifluoroethylene) and/or poly(chlorotrifluoroethylene-co-vinylidene fluoride) with PEO/PCL copolymers and/or polyurethanes such as polyurethane having monomeric units derived from hexamethylenediisocyanate, 1,4-butanediol and poly(ethylene oxide), or polyurethane having monomeric units derived from linear polycarbonated diol, isophorone diisocyanate, and isophorone diamine.

9. Material according to any one of the preceding claims wherein the polymer nanofibre material has a translucence of at least 60 % for at least one wavelength in the range from 400 nm to 900 nm wherein the translucence is determined on the basis of transmittance measurements in the visible light region using a standard two-beam UV-vis spectrometer on samples of polymeric nanomembranes with thickness of 0.03 mm in a cuvette filled with distilled water, the same solution in a second cuvette is used as reference.

10. Material according to any one of the preceding claims wherein the singlet-oxygen-producing photosensitizer is selected from the group of: free base tetraphenylporphyrin (TPP), its zinc or magnesium derivative, zinc or aluminium phthalocyanine (ZnPc or AlPc), acridine orange (AO), toluidine blue (TB), crystal violet (CV), methylene blue (MB), malachite green (MG), rose bengal (RB), hypericin, hypocrellin A, naphthalocyanines with the absorption in the near infrared region; and/or
wherein the NO radical photodonor is selected from the group of: 1-amino-3-(trifluoromethyl)-4-nitrobenzenamine, 4-(N-(aminopropyl)-3-(trifluoromethyl)-4-nitrobenzenamine)-7-nitrobenzofurazan, dimethylnitrobenzene, 4-nitro-3-(trifluoromethyl)aniline, 6-nitrobenzo[a]pyrene, 1-[(2'-nitrophenyl)methoxy]-2-oxo-3,3-diethyl-1-triazene, 2,6-dimethylnitrobenzene, 4-(4,4-difluoro-2,6-diiodo-1,3,5,7-tetramethyl-4-bora-3a,4a-diaza-s-indacen-8-yl)-N-(3-((4-nitro-3-(trifluoromethyl)phenyl)(nitroso)amino)propyl)butanamide.

11. Material according to any one of the preceding claims, comprising a singlet-oxygen-producing photosensitizer, which further comprises an inorganic or organic iodide, preferably the iodide is selected from potassium iodide, sodium iodide, magnesium iodide and calcium iodide and tetraethylammonium iodide.

12. Material according to any one of the preceding claims, which contains a combination of several singlet-oxygen-producing photosensitizers, wherein the absorption spectra of the individual singlet-oxygen-producing photosensitizers overlap in less than 70 % of wavelength, more preferably overlap in less than 50 % of wavelength, even more preferably overlap in less than 30 % of wavelength, yet more preferably do not overlap; or
which contains a combination of several NO radical photodonors, wherein the absorption spectra of the individual NO radical photodonors overlap in less than 70 % of wavelength, more preferably overlap in less than 50 % of wavelength, even more preferably overlap in less than 30 % of wavelength, yet more preferably do not overlap; or
which contains a combination of singlet-oxygen-producing photosensitizer(s) and NO radical photodonor(s), wherein the absorption spectra of singlet-oxygen-producing photosensitizer(s) and NO radical photodonor(s) overlap in less than 70 % of wavelength, more preferably overlap in less than 50 % of wavelength, even more preferably overlap in less than 30 % of wavelength, yet more preferably do not overlap.

13. Material according to any one of the preceding claims, which further contains magnetic or magnetizable particles which are encapsulated in the nanofibers or adsorbed on the nanofibers, wherein preferably the magnetic or magnetizable particles are particles on the basis of maghemite or magnetite stabilized by polyethyleneimine.

14. Method for manufacturing of the material according to any one of the preceding claims, which comprises the following steps:
- providing a solution or dispersion of a mixture of hydrophilic polymer(s) and hydrophobic polymer(s), and/or copolymer(s) with hydrophilic and hydrophobic domains;
- adding at least one singlet-oxygen-producing photosensitizer and optionally further at least one I₃⁻/I₂ indirect photodonor, and/or at least one NO radical photodonor to the solution or dispersion;
- producing nanofibers from the said solution or dispersion, preferably on a base material or substrate, preferably by electrospinning or centrifugal spinning;
- optionally subjecting the produced nanofibers to chemical or physical treatment.

15. Use of the material according to any one of the claims 1-13 as antimicrobial wound dressings, antimicrobial cosmetic facial masks, self-disinfecting face masks or respirators, self-disinfecting filters for filtration of gases or liquids, self-disinfecting textile and products made thereof, self-disinfecting packaging material or protective agriculture foils.
